# EUROPEAN PATENT APPLICATION

(11) **EP 3 088 334 A2**
(43) Date of publication of application: **02.11.2016**
(21) Application number: 16167835.4
(22) Date of filing: 01.05.2016
(51) Int. Cl.: B65F 1/00, A61L 2/232, A61L 2/238

(54) **NANO SILVER FOR NEUTRALIZING THERMOPLASTIC BAG AND SHOE MALODORS**

(30) Priority: 30.04.2015 US 201562155105 P
(71) Applicant: International Flavors & Fragrances Inc., New York, NY 10019 (US)
(72) Inventor: Brain, Joseph, Colts Neck, NJ New Jersey 07722 (US); Piatek, Bozena M, Middletown, NJ New Jersey 07748 (US); Geno, Jason, Morganville, New Jersey 07751 (US)
(74) Representative: Lawrence, John

(57) **Abstract**

A thermoplastic bag coated on at least a portion of its interior surface with nano silver is provided. Also described is a pressurized aerosol composition containing nano silver and methods of preparing a malodor neutralizing thermoplastic bag and controlling or reducing shoe odor using nano silver.

## Description

### Background

Plastic trash bags have long been used to line trash receptacles. The trash bags encourage sanitary conditions by preventing the refuse from contacting the receptacle. Trash bags also provide a convenient way to remove trash from a receptacle for transport or disposal. Because trash often contains food scraps and other malodor producing items, attempts have been made to produce fragranced trash bags to hide the malodors produced by the bag contents. See WO 2013/028362, WO 2011/084492, WO 2012/078626 and WO 2013/154899.

Nano silver has been suggested for use in deodorizing clothes, surfaces in kitchens, and garbage cans. See Luoma (September 2008) Silver Nanotechnologies and the Environment: Old Problems or New Challenges, PEN 15, page 12; and Nanosid S Product Brochure (2014), page 11. Further, Chinese Patent Application Publication CN 202807567 teaches a garbage can that includes, on its sides and top, plastic filters containing nano silver to remove malodors. Moreover, WO 02/055115 suggests the use of a salt of silver or a silver oxide in an odor reducing composition, which may be placed in contact with or incorporated into garbage bags.

### Summary of the Invention

This invention is a thermoplastic bag having an interior and exterior surface, wherein at least a portion of the interior surface of the bag has a coating of nano silver. In some embodiments, the nano silver is a spherical particle having a diameter in the range of 0.1 nm to 100 nm.

This invention also provides a method for preparing a malodor neutralizing thermoplastic bag by providing a web of thermoplastic material having an interior surface and exterior surface; applying a coating of nano silver to at least a portion of the interior surface; folding the web; and sealing the web to form a malodor neutralizing thermoplastic bag. In certain embodiment, a drawstring is attached to the web.

This invention further provides a liquid or aerosol composition of nano silver, which is contained in an aerosol spray container or pump spray dispenser for neutralizing shoe malodor. In some embodiments, the aerosol composition further includes a propellant for expelling contents of the aerosol spray container when pressure is released. In other embodiments, the composition further includes a thickening agent. In further embodiments, the amount of nano silver is in the range of from about 10 to 100 ppm. In particular embodiments, the nano silver is a spherical particle having a diameter in the range of 0.1 nm to 100 nm. A method for controlling or reducing shoe odor using the spray is also provided.

### Brief Description of the Drawings

Figure 1 shows the malodor intensity (LMS, Labeled Magnitude Scale) of nano silver coated garbage bag as compared to commercial garbage bags without malodor control (ctrl) and with malodor control including ODOR BLOCK Kitchen Bags (HEFTY) and ODORSHIELD Kitchen Bags (GLAD).
Figure 2 shows the malodor intensity (LMS 0-5) of nano silver coated garbage bag as compared to commercial garbage bags without malodor control (ctrl) and with malodor control including ODOR BLOCK Kitchen Bags (HEFTY). Results are an average of two independent experiments.
Figure 3 shows a comparison of the malodor intensity (LMS 0-5) reducing activity of nano silver (NS) compared to colloidal silver (CS).
Figure 4 shows mass spectrometry analysis of headspace above a control thermoplastic bag, a thermoplastic bag coated with colloidal silver, and a thermoplastic bag coated with nano silver. Onions were placed in the bags and the volatile compounds produced by the onions were measured.
Figures 5A and 5B show the size distribution by number of the N9 nano silver particles used in the Examples. Figures 5A and 5B represent two separate experimental determinations of five runs each. The average is indicated with an arrow.
Figure 6 shows the intensity of malodor in shoes when sprayed with a 55 ppm solution of nano silver as compared to a control (water). Data are the average of five days of scoring.

### Detailed Description of the Invention

It has now been found that a suspension of nano silver, when applied to the inside of a plastic garbage bag or shoe controls malodors, in particular sulfur- and amine-based malodors. By way of illustration, analytical and sensory evidence indicate that when nano silver is applied to the inside portion of a plastic garbage bag it scavenges/eliminates malodor caused by fresh onion or single malodor components such as dibutyl sulfide, hydrogen sulfide, butyl amine and amyl amine. Thus, nano silver has now been shown to effectively neutralize malodor.

Accordingly, the present invention features a thermoplastic bag having an inside surface and an outside surface, wherein at least a portion of the inside or interior surface is coated with nano silver, and a method for preparing the thermoplastic bag for malodor control or neutralization. Further, the present invention provides a liquid or aerosol composition containing nano silver and method for neutralizing shoe malodor using the same.

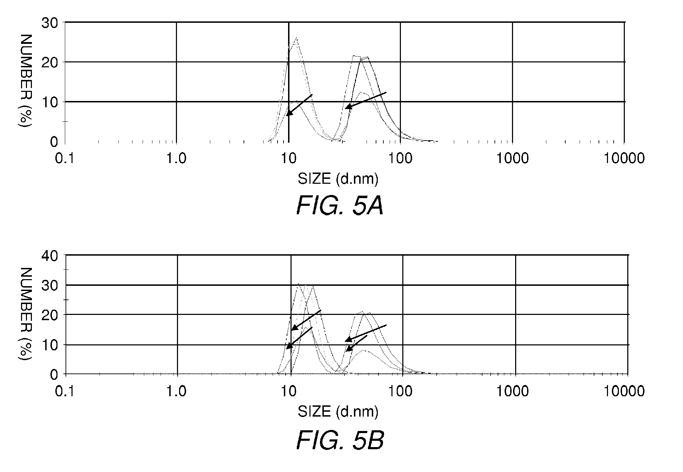

Nano silver refers to particulate silver having a diameter in the range of 0.1 nm to 100 nm, preferably in the range of 0.1 to 50 nm or most preferably in the range of 0.1 to 10 nm. In certain embodiment, the nano silver particles have a spherical shape, wherein the majority of particles (>55%) have an equivalent diameter in range of 0.2 to 4 nm or 0.5 to 6 nm. See, *e.g.,* WO 2012/059944 and WO 2012/059943. See also Figures 5A and 5B.

"Malodor" refers to compounds generally offensive or unpleasant to most people. Malodors are usually caused by particularly odorous substances which are, however, frequently only present in trace amounts. Such substances include, for example, nitrogen-containing compounds such as ammonia and amines, heterocyclic compounds such as pyridines, pyrazines, indoles, etc. and sulfur-containing compounds such as hydrogen sulfide, mercaptans, sulfides, acidic compounds such as acetic acid, butyric acid and fatty acids, and aldehyde compounds such as acetaldehyde and formaldehyde. In particular embodiments, sulfur-based malodors are controlled in accordance with the present invention.

"Neutralize," "neutralization" or "neutralizing" refers to the ability of a compound or product to reduce, control or eliminate malodorous compounds. Odor neutralization may be partial, affecting only some of the malodorous compounds in a given context, or affecting only part of a malodorous compound. A malodorous compound may be neutralized by chemical reaction resulting in a new chemical entity, by sequestration, by chelation, by association, or by any other interaction rendering the malodorous compound less malodorous or non-malodorous. In accordance with the present invention, the malodor is neutralized by a chemical reaction (Scheme 1) between metallic silver and the malodor:

2Ag (s) + H₂S => Ag₂S + H₂

Neutralization is distinguishable from odor masking or odor blocking by a change in the malodorous compound, as opposed to a change in the ability to perceive the malodor without any corresponding change in the condition of the malodorous compound. Malodor neutralization provides a sensory and analytically measurable *(e.g.,* gas chromatograph) malodor reduction. Thus, if a malodor control composition delivers genuine malodor neutralization, the composition will reduce malodors in the vapor and/or liquid phase. See, *e.g*., Example 2.

A thermoplastic bag of the invention can be prepared from films of materials, *e.g*., hydrophobic polymers, which are not derivatized by actives, such as fragrance components and malodor control agents. Useful materials in the inventive films include but are not limited to thermoplastic polyolefins, including polyethylene and copolymers thereof and polypropylene and copolymers thereof. Suitable polyethylenes include high density polyethylene, medium density polyethylene, low density polyethylene, very low density polyethylene, and linear low density polyethylene.

The olefin-based polymers include the most common ethylene or propylene based polymers such as polyethylene, polypropylene, and copolymers such as ethylene vinylacetate (EVA), ethylene methyl acrylate (EMA) and ethylene acrylic acid (EAA), or blends of such polyolefins. Other examples of polymers suitable for use as films include elastomeric polymers. Suitable elastomeric polymers may also be biodegradable or environmentally degradable. Suitable elastomeric polymers for the film include poly(ethylene-butene), poly(ethylene-hexene), poly(ethylene-octene), poly(ethylene-propylene), poly(styrene-butadiene-styrene), poly(styrene-isoprene-styrene), poly(styrene-ethylenebutylene-styrene), poly(ester-ether), poly(ether-amide), poly(ethylene-vinylacetate), poly(ethylene-methylacrylate), polyethylene-acrylic acid), poly(ethylene butylacrylate), polyurethane, poly(ethylene-propylene-diene), ethylenepropylene rubber. Rubber-like polymers may also be employed and are generally referred to herein as metallocene polymers or polyolefins produced from single-site catalysts. The most preferred catalysts are known in the art as metallocene catalysts whereby ethylene, propylene, styrene and other olefins may be polymerized with butene, hexene, octene, etc., to provide elastomers suitable for use in accordance with the principles of this invention, such as poly(ethylene-butene), poly(ethylene-hexene), poly(ethylene-octene), poly(ethylene-propylene), and/or polyolefin terpolymers thereof. It can be suitable to blend into the resin a suitable amount of a cling agent, such as polyisobutylene, to control the level of lamination during the lamination process.

In some embodiments, the film compositions may differ slightly according to their use or where there is a multilayer film, the film layers may differ from each other. For example, the film layers may have different strength or barrier properties, or properties designed for better sealing.

The webs or films of the invention can be made by a conventional flat or tubular cast extrusion or coextrusion, or other suitable process such as a blown film process to produce monolayer, bilayer, trilayer or multilayer films. If desired for a given end use, these films can be oriented by tenterframe, or other suitable process. They can thereafter optionally be annealed. The films of the present invention are typically produced by the blown film or cast film process. The blown or cast film is formed by extrusion. For the blown film process, the film can be collapsed to double the plies of the film or the film can be cut and folded or cut and unfolded. The extruder is a conventional one using a die, which will provide the desired gauge. Some useful extruders are described in U.S. Pat. Nos. 4,814,135; 4,857,600; 5,076,988; 5,153,382; each of which are incorporated herein by reference. The gauge of the films of interest here can be in the range of about 0.1 to about 10 mils, suitably from about 0.2 to about 4 mils, and suitably in the range of about 0.3 to about 2 mils. Examples of various extruders, which can be used in producing the film of the present invention, are the single screw type modified with a blown film die and air ring and continuous take off equipment.

In accordance with the present invention, the web or film is subsequently modified to include a coating of nano silver on at least a portion of the surface that will be present on the interior or inside of the bag. The resulting thermoplastic bag will have at least 5%, 10%, 20%, 30%, 40%, 50%, 60%, 70%, 80%, 90% or 100% of the interior surface coated or covered with nano silver. The nano silver may be applied as a liquid emulsion, liquid dispersion or a liquid suspension of water and active ingredient comprising or consisting of the nano silver particles. In certain embodiments, the liquid emulsion, liquid dispersion or a liquid suspension contains about 0.5% to about 5% of nano silver particles or more preferably between 1% and 2% of nano silver particles.

A method for preparing a malodor neutralizing thermoplastic bag is also provided. This method includes the steps of providing a web or film of thermoplastic material having an interior surface and exterior surface; applying a coating of nano silver to at least a portion of the interior surface; folding the web; and sealing the web to form a thermoplastic bag. In more particular embodiments, the method includes the steps of providing a folded web having interior adjacent surfaces and exterior non-adjacent surfaces, opening the folded web and inserting one or more micro-droplet applicators between the interior adjacent surfaces of the web, using the one or more micro-droplet applicators to apply nano silver by an intermittent application of droplets using fluid pressure onto the interior surface of the folded web, closing the folded web, providing heat seals to the folded web, providing a separating means (*e.g*., perforations) along the heat seals, and rolling the folded web into a roll. See also US 2013/0202229 and WO 2014/025350 as well as US 5,890,810, US 6,488,222, US 6,379,292, US 5,967,663, incorporated herein by reference, for general approaches for producing thermoplastic bags.

The present invention also includes a draw tape bag. Draw tape bags include two layers of plastic film, which are sealed on three sides and open on the remaining side to form an opening in the bag. A hem securing the draw tape is provided at the periphery of the open end whereby the tape is accessed through openings in the hem. By pulling the draw tape, the opening in the bag closes. Consequently, the draw tape serves as a handle whereby the bag may be grasped to be subsequently transported. The hem in a draw tape bag is formed by two layers of film which are fused together to create a hem seal. The hem seal is typically created by heating the film until it melts and then fusing the two layers together. Heat sealing operations typically create a strong bond which cannot be separated without destroying the film, otherwise known as a destructive bond. Draw tape bags and methods for making draw tape bags are described in US 4,867,735, US 4,966,059, and US 5,006,380, which are incorporated herein by reference. In one embodiment, the draw tape may include a single layer. In one embodiment, the draw tape may include a first exterior layer, a second exterior layer, and at least one core layer disposed between the exterior layers as described in US 2010/0172602.

The nano silver lined or coated thermoplastic bag of the invention can be unscented or scented with one or a mixture of fragrances. Moreover, the bag may include a fragrance release inhibitor in the same or different area as the fragrance and nano silver. A typical fragrance release inhibitor is titanium dioxide. Additional fragrance release inhibitors include starch, clays and nanoclays, talc, and microcapsules.

This invention also provides a composition and method for neutralization of shoe malodor by spraying or applying nano silver to the interior and/or exterior of a shoe, *e.g.,* a boot, sandal, clog, tennis shoe, cleat, loafer, or any other form of footwear. Accordingly, a liquid or aerosol composition containing nano silver is also encompassed by this invention. In certain embodiments, the liquid or aerosol composition contains up to 1000 ppm nano silver. In other embodiment, the composition contains between 10 and 100 ppm nano silver, or more preferably between 20 and 80 ppm nano silver, or most preferably between 45 and 65 ppm nano silver.

For the purposes of the present invention, a liquid composition is a formulation of nano silver suitable for being dispensed via a pump spray dispenser (*e.g*., a spray container, an atomizer or the like), which sprays or mists the nano silver. Examples of such pump spray dispensers are known in the art. See, *e.g.,* US 5,183,187 and US 5,464,129, incorporated herein by reference.

An aerosol composition of this invention is intended to refer to a formulation of nano silver suitable for being dispensed via in an aerosol spray container or pressure bottle. In this respect, the nano silver formulation includes a propellant, which is a gas for expelling the nano silver when pressure is released. The gas or gases should have a sufficiently high vapor pressure in the aerosol canister to pressurize the contents of the canister to expel the composition from the aerosol canister. Suitable aerosol spray containers are known in the art. See, *e.g.,* US 5,921,439 and US 3,712,515, incorporated herein by reference.

Suitable propellants include ethers such as dimethyl ether (DME); and aliphatic hydrocarbons such as the C₃ to C₅ hydrocarbons, including propane, butane, n-butane, isobutane or mixtures thereof. Such propellants, individually, have vapor pressures ranging from about 17 to about 100 psig at 70°F, preferably from about 25 to about 50 psig at 70°F. The amount of propellant in the aerosol can range from about 10 to about 90% (wt), preferably from about 40 to about 85%, more preferably from about 65 to about 80%, most preferably about 70 to about 77%.

The nano silver of the liquid or aerosol composition can be provided in a solvent, which can be any substance capable of carrying and/or maintaining the nano silver and other ingredients in the composition in a substantially uniform mixture or suspension for uniform expulsion and dissipation from a container to the target shoe. Suitable solvents can include water; and organic solvents capable of evaporating from the shoe surface such as C₁ to C₃ alcohols, including methanol, ethanol, propanol and isopropanol. The solvent is used in amounts effective to carry and/or maintain the nano silver and other ingredients in the composition in a substantially uniform mixture or suspension in the presence or absence of a pressurizing propellent. The amount of solvent in the composition can be from about zero (0) to about 80 percent, preferably from about 4 to about 50 percent, more preferably from about 5 to about 20 percent. Without the propellant, the amount of solvent in the composition can range from about zero (0) to about 80% by weight, more preferably from about 20 to about 75%.

Optionally, a thickening agent can also be added to the composition to thicken the contents of the composition, including the nano silver, the solvent and any other ingredients, to maintain more uniformly or homogenously the ingredients in the composition. Suitable thickening agents include Bentone® thickener which is an organically modified hectorite (marketed by Rheox Inc. of Hightstown, NJ), fatty alcohols such as cetyl, lauryl, stearyl, and the like; soaps such as sodium stearate, sodium myristate and the like, bentonite, cellulosic ethers such as methyl cellulose, sodium cellulose glycollate (sodium carboxymethyl cellullose), silica gel, alumina gel or mixtures thereof. A thickening agent may optionally be included in the composition in an amount ranging from about 0 to about 1 percent, preferably from about 0.1 to about 1 percent, more preferably from about 0.2 to about 1 percent. Without the propellant, the amount of thickening agent in the composition can range from about 0.1 to about 5% by weight, more preferably from about 0.2 to about 3%.

Optionally, a fragrance (an aromatic compound) can be added to the composition to impart an aesthetically pleasing aroma to the composition and to mask any shoe odors. Typical fragrances include aromatic materials extracted from botanical sources (*i.e*., rose petals, gardenia blossoms, jasmine flowers, etc.) which can be used alone or in any combination. Alternatively, alcoholic extracts may be prepared for compounding fragrances. The fragrance may also be encapsulated. One or more fragrances can optionally be included in the composition in an amount ranging from about 0 to about 5 percent, preferably from about 0.01 to about 5 weight percent, also preferably about 0.1 to about 3 percent, more preferably from about 0.2 to about 2.5 percent. Without the propellant, the amount of fragrance in the composition can range from about zero (0) to about 15% by weight, preferably from about one to about 11%.

The following non-limiting examples are provided to further illustrate the present invention.

### Example 1: Control of Onion Malodor

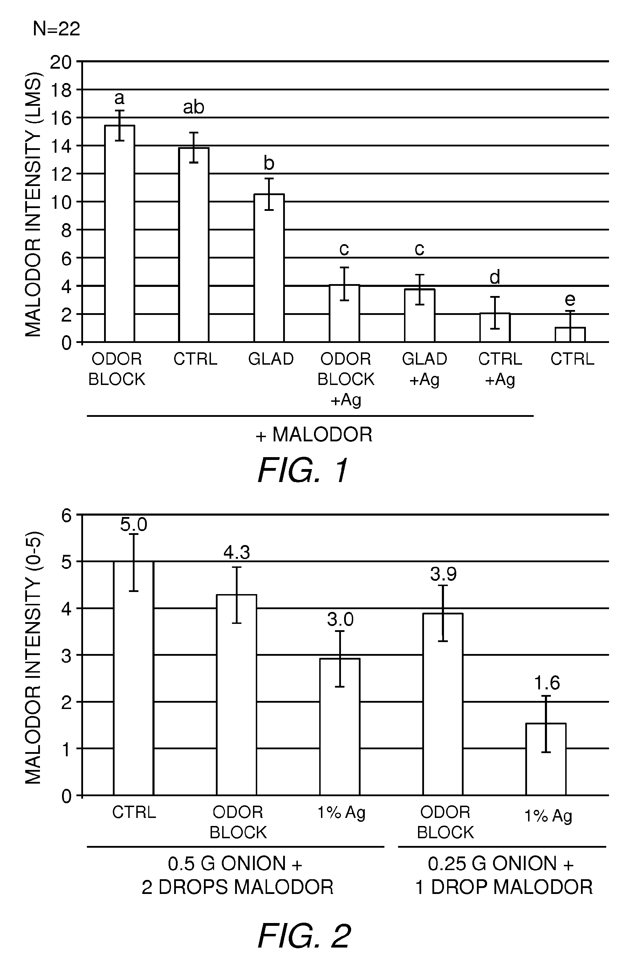

Nano silver (Pure Silver Super) was supplied by N9 World Technologies Pvt Ltd (Bangalore, India). The Pure Silver Super was a 1100 ppm solution, which was applied to the interior surface of a kitchen garbage bag which was then allowed to dry. Fresh onion was placed inside the garbage bag as a malodor source and left to stand overnight. A panel of test subjects analyzed the malodor intensity of the nano silver lined garbage bag as compared to commercial garbage bags without malodor control (control) and with malodor control including ODOR BLOCK Kitchen Bags (HEFTY) and ODORSHIELD Kitchen Bags (GLAD). This analysis indicated that the nano silver lined garbage bag provided a significant reduction in malodor intensity (Figure 1).

### Example 2: Control of Onion and Garbage Malodor

Nano silver (Pure Silver Super) was supplied by N9 World Technologies Pvt Ltd (Bangalore, India). A 1% solution of nano silver was applied to the interior surface of a kitchen garbage bag which was then allowed to dry. Fresh onion (0.25 g or 0.5 g) and garbage malodor (1 drop or 2 drops) was placed inside the garbage bag as a malodor source and left overnight. A panel of test subjects analyzed the malodor intensity of the nano silver lined garbage bag as compared to a commercial garbage bag without malodor control (control) and with malodor control including ODOR BLOCK Kitchen Bags (HEFTY). This analysis indicated that the nano silver lined garbage bag provided a significant reduction in malodor intensity (Figure 2).

### Example 3: Malodor Control with Different Forms of Silver

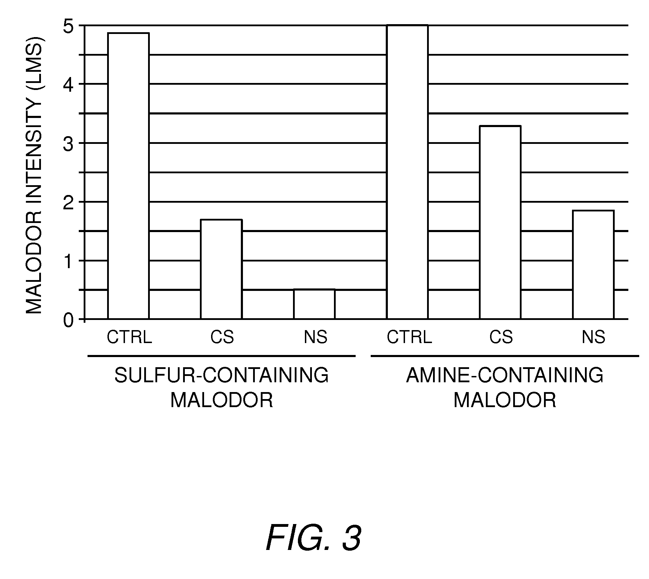

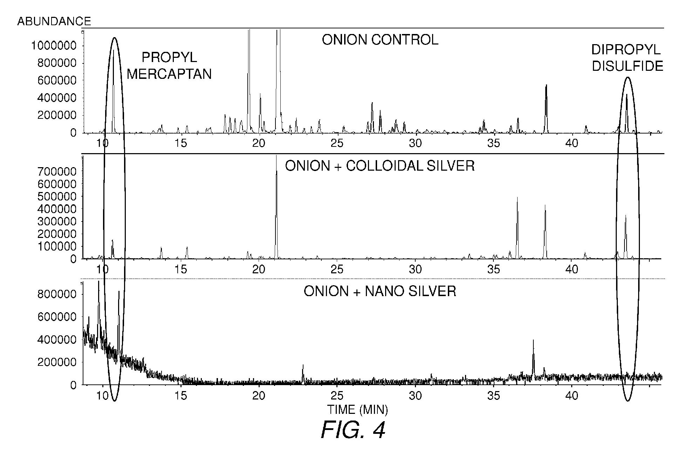

To determine the effect of different forms of silver on malodor control, nano silver and colloidal silver were applied to the interior surface of garbage bags which were then allowed to dry and control of sulfur and amine malodors was analyzed. This analysis indicated that the olfactive malodor scores of colloidal silver were better than the control without silver, but not as good as nano silver in control sulfur and amine malodors (Figure 3). Headspace analysis further confirmed that nano silver reduced the levels of propyl mercaptan and dipropyl disulfide produced by at least 1000-fold compared to the control, whereas colloidal silver only provided a 10-fold reduction compared to the control (Figure 4).

### Example 4: Control of Shoe Malodor

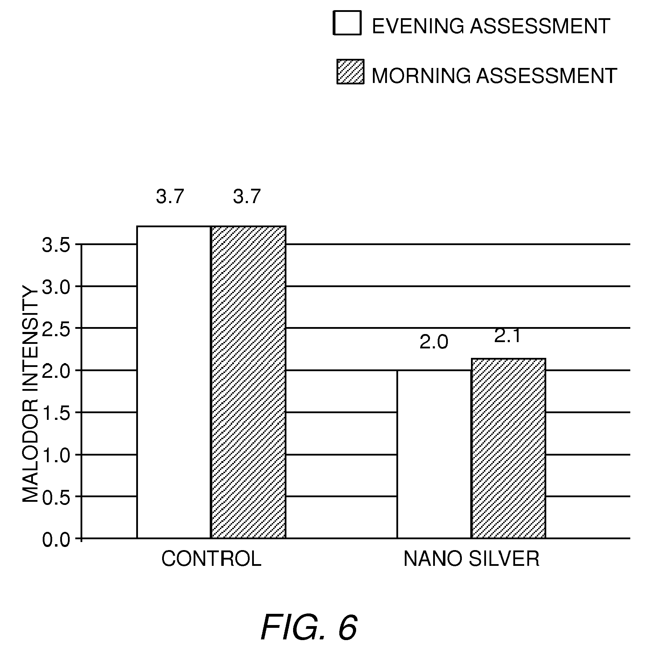

Malodor control in shoes was also assessed. A panel of subjects was instructed to pick a pair of shoes that could be worn for an entire week and with socks. On the first evening of the test, subjects were asked to apply three sprays to the left shoe with a bottle labeled "left shoe," which, unbeknownst to the subject, contained the water control, and three sprays to a right shoe with a bottle labeled "right shoe," which contained a 55 ppm solution of nano silver. In accordance with the instructions, the spray was to be evenly applied so that the mist would go down to the toe area as well as the heel area of the shoe. Shoes were allowed to dry overnight. On the morning of the second day of the test, malodor of the left and right shoes was evaluated on a 0 to 5 scale with 0 being no malodor and 5 being strong malodor. After being worn all day, shoes were again evaluated on the evening of the second day using the same 0 to 5 scale. The steps of spraying in the evening and evaluating malodors in the morning and evening were repeated for an additional 4 days (*i.e*., a total of 5 days of morning and evening evaluations). Based upon the results of this test (Figure 6), it was demonstrated that malodor levels were lower in shoes sprayed with nano silver.

### Example 5: Shoe Deodorant Compositions

*Formulations Without Thickener* (Table 1). To an explosion proof jacketed batch tank, mix about two-thirds of the ethyl alcohol and the sodium bicarbonate. Pass the mixture through a colloid mill. Mix in the remaining ethyl alcohol, the 55 ppm nano silver and the fragrance to form a concentrate. Pass the concentrate through a colloid mill and fill an aerosol can with 23% concentrate and 77% isobutene propellant.

**TABLE 1**

| Ingredient | % wt/wt basis in aerosol (with propellant) | % wt/wt basis in concentrate (no propellant) |
|---|---|---|
| Isobutane Propellant | 77.00 | 0.00 |
| Ethyl Alcohol | 17.22 | 75.0 |
| Sodium Bicarbonate | 1.28 | 5.5 |
| Fragrance | 0.25 | 1.0 |

*Formulations With Thickener* (Table 2). The formulations are prepared as described above, except that BENTONE is added to the concentrate and the percentages of the remaining ingredients are modified.

**TABLE 2**

| Ingredient | % wt/wt basis in aerosol (with propellant) | % wt/wt basis in concentrate (no propellant) |
|---|---|---|
| Isobutane Propellant | 77.00 | 0.00 |
| Ethyl Alcohol | 6.0 | 43.5 |
| Sodium Bicarbonate | 4.0 | 17.5 |
| BENTONE | 0.5 | 2.1 |
| Fragrance | 2.5 | 10.9 |

## Claims

1. A thermoplastic bag comprising an interior and exterior surface, wherein at least a portion of the interior surface of the bag comprises a coating of nano silver.

2. The thermoplastic bag of claim 1, wherein the nano silver comprises a spherical particle having a diameter in the range of 0.1 nm to 100 nm.

3. A method for preparing a malodor neutralizing thermoplastic bag comprising providing a web of thermoplastic material having an interior surface and exterior surface; applying a coating of nano silver to at least a portion of the interior surface; folding the web; and sealing the web to form a malodor neutralizing thermoplastic bag.

4. The method of claim 3, wherein a drawstring is attached to the web.

5. A liquid or aerosol composition of nano silver, which is contained in an aerosol spray container or pump spray dispenser for neutralizing shoe malodor.

6. The liquid or aerosol composition of claim 5, further comprising a propellant for expelling contents of the aerosol spray container when pressure is released.

7. The liquid or aerosol composition of claim 5, or claim 6, further comprising a thickening agent.

8. The liquid or aerosol composition of claim 5, or claim 6, or claim 7, wherein the amount of nano silver is in the range of from 10 to 100 ppm.

9. The liquid or aerosol composition of claim 5, or any of claims 6 to 8, wherein the nano silver comprises a spherical particle having a diameter in the range of 0.1 nm to 100 nm.

10. A method of neutralizing shoe malodor comprising contacting a shoe with the liquid or aerosol composition of claim 5, or of any of claims 6 to 9, thereby neutralizing shoe malodor.
